# EUROPEAN PATENT SPECIFICATION

(11) **EP 1 896 121 B1**
(45) Date of publication and mention of the grant of the patent: **07.11.2012**
(21) Application number: 06770479.1
(22) Date of filing: 16.05.2006
(51) Int. Cl.: A61M 39/26

(54) **ANTI-DRAWBACK MEDICAL VALVE**
MEDIZINISCHES ANTIRÜCKLAUFVENTIL
VALVE MEDICALE ANTI-REFOULEMENT

(30) Priority: 16.05.2005 US 681275 P
(43) Date of publication of application: 12.03.2008
(73) Proprietor: Nypro Inc., Clinton, MA 01510-2005 (US)
(72) Inventor: COTE,SR., Andrew, L., Merrimack, NH 03054 (US); NEWTON, Brian, L., Woonsocket, RI 02895 (US); GANEM, Charles, F., Cape Neddick, ME 03902 (US)
(74) Representative: Greene, Simon Kenneth
(86) International application number: PCT/US2006/019055
(87) International publication number: WO 2006/124981

(56) References cited:
- US-A- 5 006 114
- US-A- 5 049 128
- US-A- 5 730 418
- US-A1- 2004 073 171
- US-B1- 6 228 069
- US-B1- 6 245 048

## Description

### Technical Field

The invention generally relates to medical valves and, more particularly, the invention relates to medical valves that substantially eliminate fluid drawback.

### Background Art

In general terms, medical valving devices often act as a sealed port that may be repeatedly accessed to non-invasively inject fluid into (or withdraw fluid from) a patient's vasculature. Consequently, a medical valve permits the patient's vasculature to be freely accessed without requiring such patient's skin be repeatedly pierced by a needle.

Medical personnel insert a syringe into the proximal port of a properly secured medical valve to inject fluid into (or withdraw fluid from) a patient. Once inserted, the syringe may freely inject or withdraw fluid to and from the patient. Problems can arise, however, when the syringe is withdrawn. Specifically, a back pressure (i.e., a proximally directed pressure) produced by the withdrawing syringe undesirably can cause blood to be drawn proximally into a catheter attached to the valve, or into the valve itself. In addition to coagulating and impeding the mechanical operation of the valve, blood in the catheter or valve also compromises sterility.
A prior art valve addressing this problem is taught by US2004/073171. It shows the combination of features defined in the preamble of claim 1. Other valves with similar functions are taught by US 6,245,048, US6,228,069, US5,006,114, US 5,730,418 and US 5,049,128.

### Summary of the Invention

The invention is defined in claim 1. In accordance with the invention, the medical valve produces no greater than a negligible drawback or positive push during withdrawal of a nozzle. To those ends, the medical valve includes a housing having an interior forming an inlet and an outlet. A longitudinally movable member is secured within the interior of the housing. External to the movable member and within the interior housing is a fluid path. Movement of the movable member controls fluid flow between the inlet and the outlet via the fluid path. As noted above, the outlet produces no greater than a negligible drawback or positive push during withdrawal of a nozzle.

In accordance with the invention, the movable member is rigid. The outlet produces no greater than a negligible drawback or positive push during attachment of the nozzle. The valve may further include at least one seal between the housing and the movable member.

The movable member has a first position that permits fluid flow from the inlet to the outlet via the fluid path, and a second position that prevents fluid flow from the inlet to the outlet via the fluid path. The movable member has a substantially static shape transitioning between the first position and the second position. The fluid path has a volume that is substantially the same in both the first position and the second position. The valve may further include a biasing member for biasing the movable member toward the towards the second position. The biasing member may be a spring. The biasing member includes an air vent, which may further include a hydrophobic filter. The movable member may have an end for contacting the nozzle which may include at least one of a groove and a protrusion. The end may be swabbable in the second position.

Further a method of controlling fluid flow through a medical valve is provided. The medical valve includes a housing having an inlet and an outlet. A longitudinally movable member is secured within the interior of the housing. The method includes inserting a nozzle into the inlet to cause the movable member to transition to a first position. In the first position, fluid flow is permitted between the inlet and the outlet, the fluid flow between the inlet and the outlet being external to the movable member. Withdrawing the nozzle from the inlet causes the movable member to transition to a second position that prevents fluid flow between the inlet and the outlet. The withdrawing includes causing the outlet to produce no greater than a negligible drawback or positive push.

Inserting the nozzle produces no greater than a negligible drawback or positive push. The movable member has a substantially static shape transitioning between the first position and the second position. The valve includes a fluid path within the interior of the valve; the fluid path having a volume that is substantially the same in both the first position and the second position. The method may further include biasing the movable member in the second position and/or venting gas between the interior of the housing and exterior of the housing.

### Brief Description of the Drawings

The foregoing features of the invention will be more readily understood by reference to the following detailed description, taken with reference to the accompanying drawings, in which:

Figure 1 schematically shows a medical valve that may be configured in accordance with illustrative embodiments;

Figure 2 schematically shows a cross-sectional view of the medical valve of figure 1 in a closed position, in accordance with an embodiment of the invention;

Figure 3 schematically shows a cross-sectional view of the medical valve of Figure 1 in an opened position, in accordance with an embodiment of the invention;

Figure 4 schematically shows an isometric view of a movable member of the medical valve of Fig.1, in accordance with an embodiment of the invention;

Figure 5 schematically shows an isometric view of an alternative embodiment of a movable member, in accordance with an embodiment of the invention;

Figure 6 schematically shows a cross-sectional view of a medical valve in a closed position, in accordance with an embodiment of the invention;

Figure 7 schematically shows a cross-sectional view of the medical valve of Figure 6 in an opened position, in accordance with an embodiment of the invention;

Figure 8 schematically shows a cross-sectional view of a medical valve in a closed position, in accordance with an embodiment of the invention;

Figure 9 schematically shows a cross-sectional view of the medical valve of Figure 8 in an opened position, in accordance with an embodiment of the invention;

Figure 10 schematically shows a cross-sectional view of a medical valve in a closed position, in accordance with an embodiment of the invention; and

Figure 11 schematically shows a cross-sectional view of the medical valve of Figure 10 in an opened position, in accordance with an embodiment of the invention.

### Detailed Description of Specific Embodiments

In illustrative embodiments, a medical valve substantially has neither a positive push nor a drawback during withdrawal and/or attachment of a nozzle. To those ends, such a valve illustratively may have a flow path that maintains a substantially static shape and thus, a substantially constant volume, during withdrawal or attachment of the nozzle. Details of illustrative embodiments are discussed below.

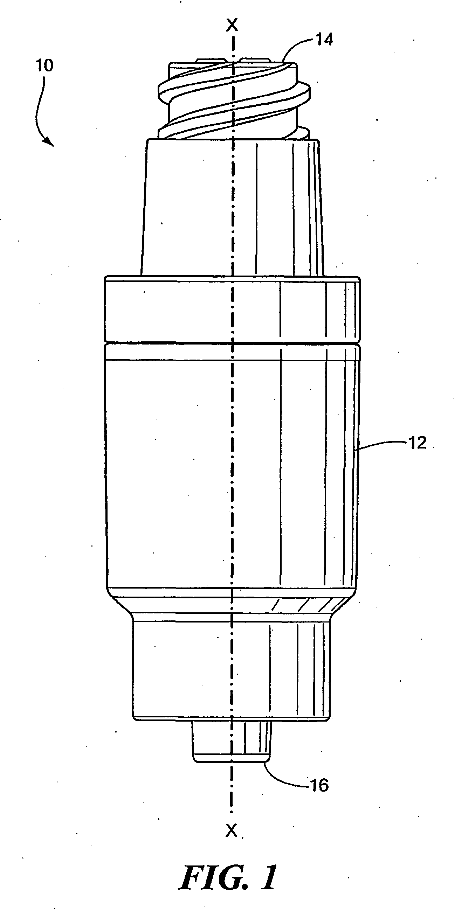

Fig.1 schematically shows a medical valve 10 that may be configured to substantially eliminate positive push and fluid drawback (a/k/a "back-flow") when a syringe or other type of nozzle is connected and/or withdrawn from it, in accordance with an embodiment of the invention. The valve 10 includes a proximal port 14 for receiving the nozzle, a valve body 12 having an internal valve mechanism (various embodiments of the valve mechanism are shown in subsequent figures) that controls fluid flow through the valve, and a distal port 16 for directing fluid between the valve 10 and a patient. The distal port may be at its location shown in Fig.1, or at a different location, such as at a location orthogonal to the longitudinal dimension of the valve 10. Although much of the discussion herein refers to the proximal port 14 as a fluid inlet, and the distal port 16 as a fluid outlet, the proximal and distal ports 14 and 16 also may be respectively used as outlet and inlet ports.

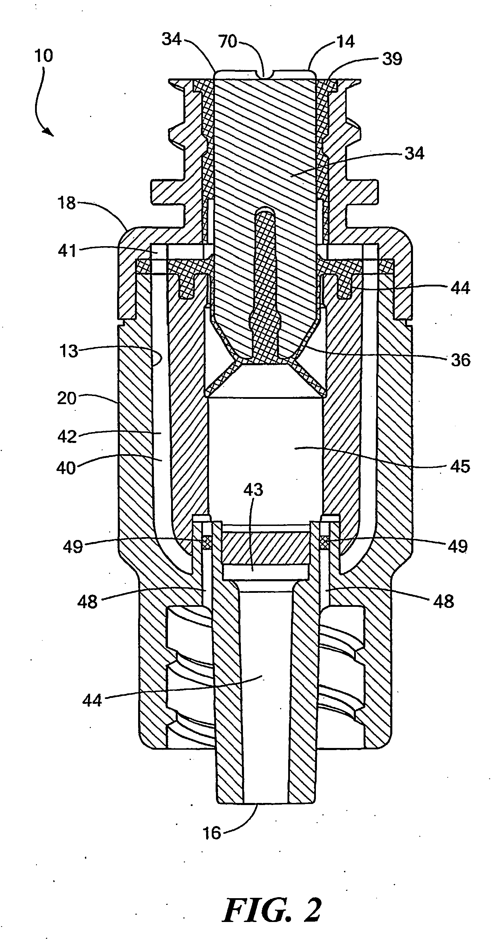

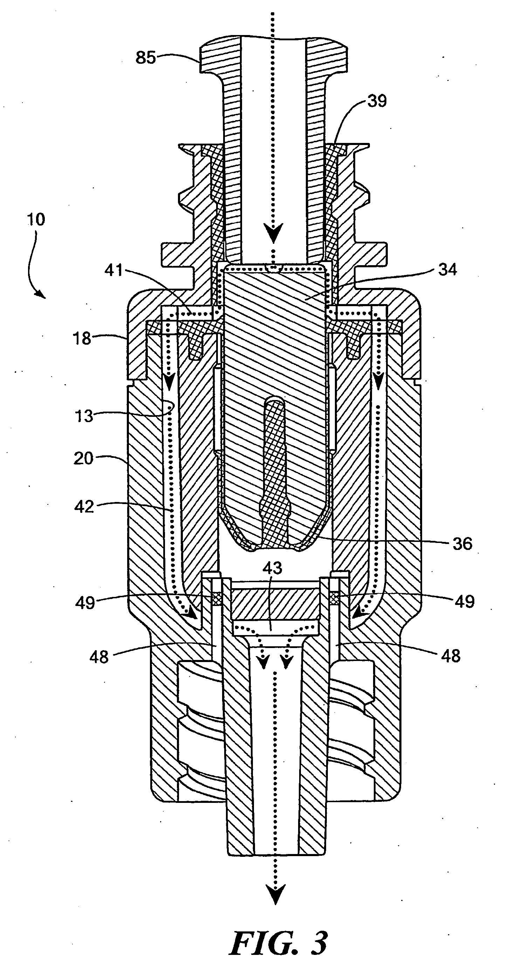

Fig. 2 schematically shows a cross-sectional view of one embodiment of the medical valve 10 (shown in Fig. 1 along line X-X) in a closed position, while Fig. 3 shows the valve 10 in an open position (e.g., activated by a luer-taper nozzle 85. The valve 10 includes a housing having an interior 13 forming the proximal and distal ports 14 and 16. The housing may be formed from inlet and outlet housing portions 18 and 20, which illustratively are formed from a hard plastic material (e.g., polycarbonate, polypropylene, or polyethylene) that are snap-fit together. For example, the housing portions 18 and 20 may be configured to snap fit together in accordance with the teachings of U.S. Patent Number 6,892,998. It should be noted that although some embodiments are discussed as being snap-fit components, various embodiments of the invention may be coupled by either snap-fit or other means, such as by an adhesive or ultrasonic welding. Accordingly, such embodiments are not intended to be limited to snap-fit components.

When coupled, the housing portions 18 and 20 form the interior 13, which is shaped to comply with the operation of its internal valve mechanism (discussed below) and permit fluid flow. The proximal port 14 illustratively is contoured to accept various types of nozzles, such as those complying with ANSI/ISO standards (e.g., luers complying with ANSI and/or ISO standards).

Secured within the interior 13 is a movable member 34 that may move, without limitation, substantially longitudinally to control fluid flow through the valve 10. The fluid, preferably in liquid form, such as blood, saline or a liquid medication, is controlled by the movable member 34 to pass through a fluid path within the interior 13 of the valve 10 between the inlet port 14 and the outlet port 16.

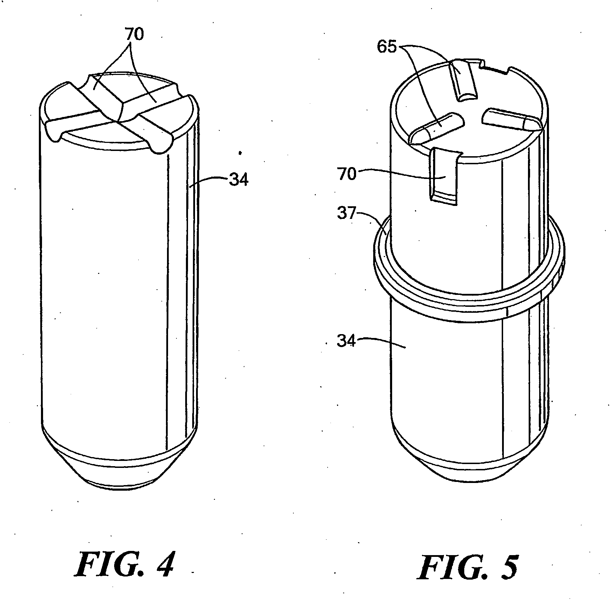

The movable member 34 is prevented from moving too far towards the distal port 16 by a spring 36 (described in more detail below). Furthermore, among other ways, the movable member 34 may be attached to the spring 36 by an interference fitting, welding and/or adhesive, such that the movable member 34 is prevented from moving too far towards the proximal port 14. In various embodiments, the movable member may include a flange 37 (see Fig. 5) that contacts the bottom edge of a luer insert 39 to prevent the movable member 34 from moving too far towards the proximal port 14.

Fig. 4 schematically shows an isometric view of the movable member 34, in accordance with an embodiment of the invention. In illustrative embodiments, the movable member 34 does not include a bore defining a portion of the fluid path. As a result, the fluid path is external to the movable member 34. The movable member may is rigid. The movable member 34 may have a substantially static shape when transitioning between the open and closed positions, and be made of, without limitation, a plastic or high durometer elastomeric material. Other materials may be used, however, so long as they can perform the functions discussed herein. The top end of the movable member may include at least one or more protrusions (e.g., ribs or bumps) and/or grooves 70. The grooves 70 or protrusions form passageways through which fluid can flow (discussed in more detail below).

Referring back to Fig. 2, when the valve 10 is in the closed position, the movable member 34 contacts the luer insert 39. The luer insert 39 acts as a seal and prevents fluid from entering or exiting the fluid path via inlet 14. The luer insert 39 may be attached to the inner wall of the housing by use of, without limitation, adhesives, a two-shot injection molding process, and/or an interference fitting (e.g., an undercut). Alternatively, the luer insert 39 may be attached to the movable member 34 such that it contacts the inner wall of the housing in the closed position to form a seal. The luer insert 39 may be made of, without limitation, a thermal set material (e.g., silicone) or a thermal plastic elastomer (e.g., kraton), and may be of the type described in U.S. Patent No. 6,585,229. In various embodiments, the luer insert 39 may be radially compressed when contacting the movable member 34.

The valve 10 also includes a flow path forming means 40 that forms the flow path through the valve and facilitates operations. Specifically, the flow path forming means 40 may include, without limitation, one or more elastomeric or plastic inserts, or may be integral to the housing (i.e., part of the housing). To facilitate fluid flow, the flow path means 40 may include, for example, a horizontal flow path 41 that leads to longitudinally extending flow paths 42. Such flow paths 42 further meet additional horizontal flow paths 43 that terminate in an outlet channel 44.

In addition to forming the flow path, the flow path forming means 40 may include attachment surfaces 44 that may work in combination with various surfaces of the valve housing to secure the spring 36. The spring 36, which may be made of, without limitation, silicone, contacts the distal end of the movable member 34 biases the movable member 34 such that the valve 10 is normally closed. In various embodiments, the spring 36 is neutral (i.e., neither compressed nor stretched) in the closed position. In other embodiments, the spring 36 is slightly stretched when the valve 10 is in the closed position.

Additionally, the flow path forming means 40 includes a central cavity 45 that permits longitudinal movement of the movable member 34. An air vent 48 may be connected to the central cavity to reduce pressure build up in the central cavity 47 due to longitudinal movement of the movable member 34. The air vent 48 may include a filter 49, such as sterilizing grade, hydrophobic vent filter that allows gas to flow through it but prevents any fluids and/or contaminants from entering the central cavity 45. The air vent 48 may allow gas to be expelled externally to the valve 10. Alternative embodiments, however, omit the air vent 48.

When the valve 10 is in the fully closed position, the top end of the movable member 34 may be flush with, or extend slightly above, the exterior inlet face of the housing. The top end 56 and the exterior inlet face thus present a swabbable surface. In other words, the top end 56 and the exterior inlet face may be easily wiped clean by any conventional means, such as with an alcohol swab. As mentioned in various ones of the above cited documents, valves having swabbable surfaces are known in the art as "swabbable valves." In other embodiments, however, the valve 10 is not a swabbable valve.

Insertion of a nozzle 85 (e.g., a luer) into the proximal port 14 forces the movable member 34 to move longitudinally to an open position, as shown in Fig. 3. When in the open position, fluid may flow from the nozzle 85, through passageways formed by at least one or more protrusions (e.g., ribs or bumps) and/or grooves 70 in the top end of movable member 34 (typically the top end of the movable member 34 is primed with fluid before the movable member 34 reaches the open position). Fluid flowing through the passageways further flows into the flow path defined by flow path forming means 40, thereby permitting fluid flow between the inlet 14 and the outlet 16. It should be noted that during all phases of the opening and closing operations, the movable member 34 illustratively traverses through a portion of the fluid path. Consequently, substantially no volume changes occur in the fluid path.

Since there is substantially no volume change in the fluid path, both when the nozzle 85 is inserted into or withdrawn from the inlet 14, the valve 10 should generate no greater than a negligible positive or negative pressure through the outlet 16. In other words, in such embodiments, the volumes of fluid forced through the outlet 16 during withdrawal and insertion are negligible. This design, which has insubstantial positive push and/or drawback, thus may be considered to have a "neutral" drawback. In practice, this embodiment can have negligible amounts in either direction (e.g., less than one microliter). For example, if the nozzle is a tapered luer, the taper may result in a negligible volume change in the fluid path when in the open position, which should not occur with a taperless nozzle.

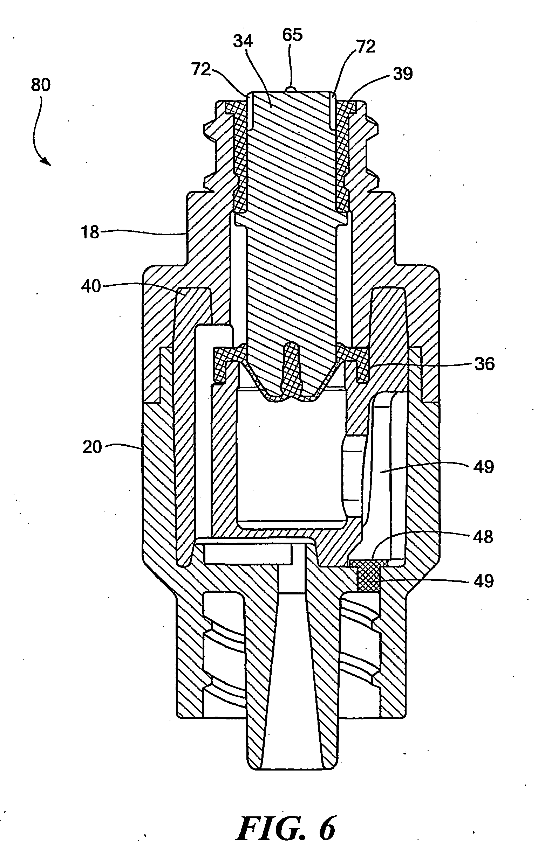

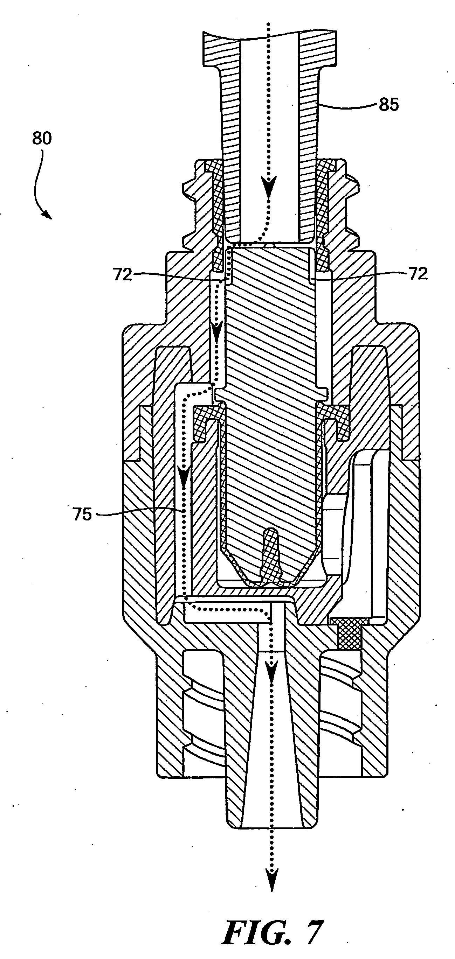

Figs. 6 and 7 schematically show cross-sectional views of the medical valve 10 configured in a different manner than shown in figures 2 and 3. Specifically, this embodiment incorporates a different embodiment of the movable member 34, which is depicted in Fig. 5. More specifically, instead of, or in addition to, at least one protrusion 65 and/or groove on the top of movable member 34, this embodiment of the movable member 34 has at least one groove 72 that extends downwardly from the top of the movable member 34. Fig. 6 shows the valve 80 in a non-activated/closed position, while Fig. 7 shows the valve 80 in an open/actuated position by a luer-taper nozzle 85. The valve 80 works in a similar manner to the valve 10 depicted in Figs. 2 and 3, with exceptions, for example, of fluid flow associated with the nozzle 85 and movable member 34, the use of a flange 37 to secure the movable member 34 within the housing, and an additional cavity 49 to further reduce pressure build up in the central cavity 47.

More particularly, upon insertion, the tip of the nozzle 85 makes contact with the movable member 34. As the movable member 34 moves longitudinally towards the distal port 16, the grooves 72 extend past the luer insert 39. Fluid from the nozzle 85 passes through the gap between the protrusions 65, and further through the grooves 72 into the fluid path forming means 40, thereby permitting fluid flow between the inlet 14 and the outlet 16, as depicted by the dotted line 75 in Fig. 7.

In various embodiments, grooves 72 may be positioned on the luer insert 39 (see Fig. 3). The grooves 72 may extend upwardly from the bottom end of the luer insert 39 a predefined distance. Upon insertion of the nozzle 85, the movable member 34 moves longitudinally towards the distal port 16, such that fluid passing through passageways formed by the protrusions and/or grooves 70 on the top of the movable member 34 can flow into the grooves 72 on the luer insert 39 and into the fluid path.

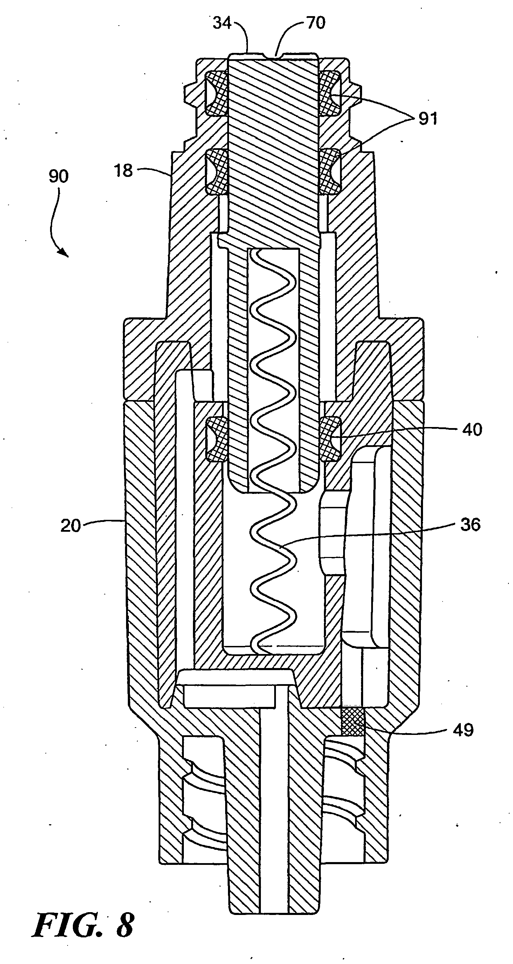

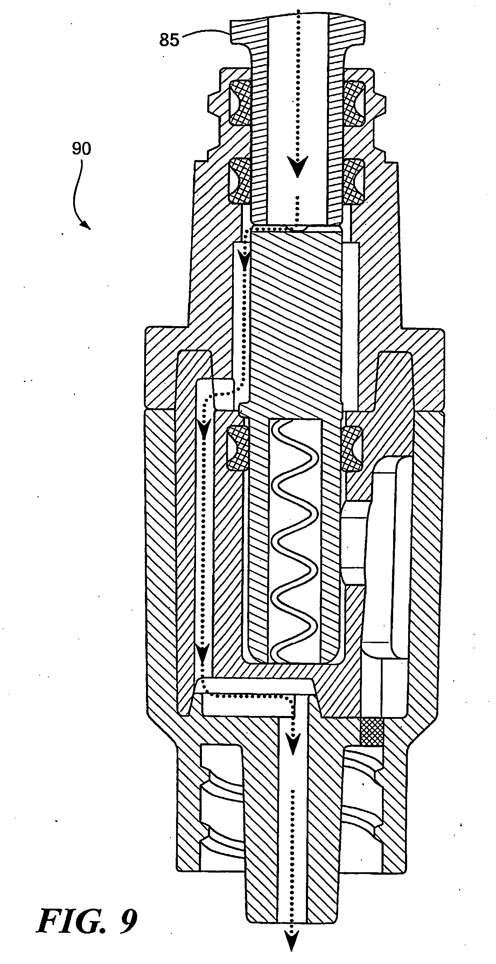

Figs. 8 and 9 schematically show cross-sectional views of another embodiment of a medical valve. Fig. 8 shows the valve 90 in a non-activated closed position, while Fig. 9 shows the valve 90 in an open position actuated by a luer-taper nozzle 85. The valve 90 works in a similar manner to the valve embodiments described above, with various exceptions. For example, the valve 90 includes a plurality of seals 91 that replace the single luer insert of Fig. 2, and also seal the distal end of the movable member. Additionally, the spring 36 is a wire compression spring that may be made, without limitation, of stainless steel or plastic. As shown in figs. 8 and 9, the movable member 34 may include a recess for securing spring 36.

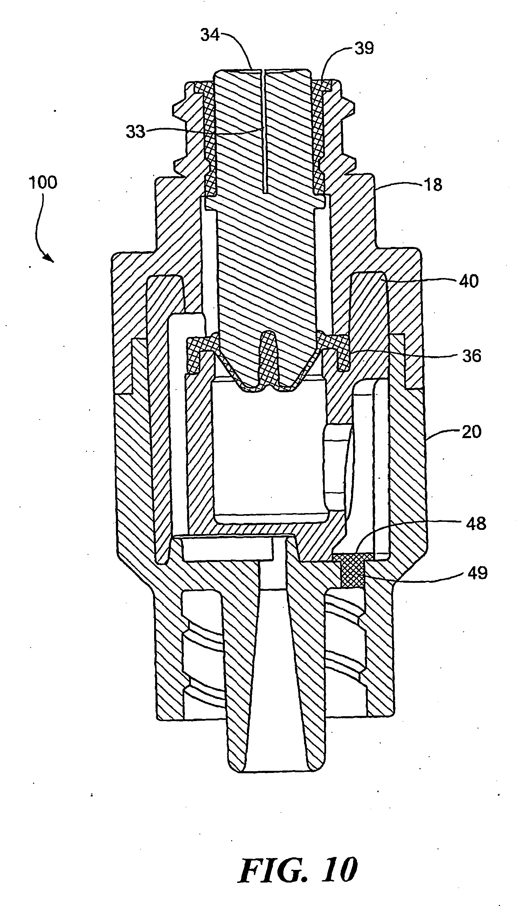

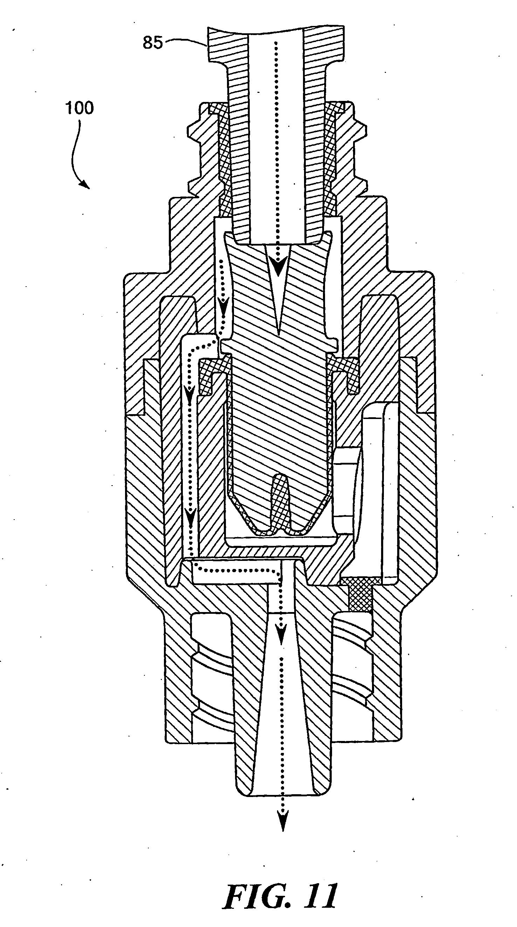

Figs. 10 and 11 schematically show cross-sectional views of still another embodiment of a medical valve. Fig. 10 shows the valve 100 in a non-activated closed position, while Fig. 11 shows the valve 100 in an open position actuated by a luer-taper nozzle 85. Instead of protrusions or grooves on the top of the movable member 34, the top end of the movable member includes a slit 33 that extends longitudinally from its proximal end (i.e., the end closest to the proximal port 14). In preferred embodiments, the slit 33 is dimensioned such that it does not extend distally beyond the luer insert 39 when the valve 10 is in the closed position. When the valve 10 is in the closed position, the post 34 contacts the luer insert 39, and the slit 33 is closed, thus preventing fluid from entering and/or exiting slit 33. In preferred embodiments, the post 34 may be radially compressed when adjacent the luer insert 39.

Insertion of a nozzle 85 (e.g., a luer) into the proximal port 14 forces the post 34 to move (radially and longitudinally) to an open position. The proximal end of the post 34 may be concave, such that the slit opens 33 when the nozzle presses against the post 34 and moves past the luer insert 39. When in that position, fluid may flow from the luer insert 39 through the slit 33 and further into the flow path forming means 40 thereby permitting fluid flow between the inlet 14 and the outlet 16, as depicted by the dotted line in Fig. 11.

Although the above discussion discloses various exemplary embodiments of the invention, it should be apparent that those skilled in the art can make various modifications that will achieve some of the advantages of the invention without departing from the scope of the invention as defined in the claims.

## Claims

1. A medical valve comprising:
a housing having an interior (13) forming an inlet (14) and an outlet (16);
a fluid path within the interior; and
a flow path forming means (40) within the interior forming at least a portion of the fluid path and having a central cavity (45); and
a rigid longitudinally movable member (34) secured within the interior of the housing, the fluid path being external to the movable member, movement of the movable member controlling fluid flow between the inlet and the outlet via the fluid path,
the movable member (34) having a first position that permits fluid flow from the inlet to the outlet via the fluid path, and a second position that prevents fluid flow from the inlet to the outlet via the fluid path,
the outlet produces no greater than a negligible drawback during withdrawal of a nozzle,
**characterised in that** the moveable member is received within the central cavity and the central cavity (45) allows movement of the longitudinally moveable member between the first position and second position such that during all phase of the opening and closing operation the moveable member (34) traverses through a portion of the fluid path which has a volume that is substantially the same in both the first position and the second position, and the outlet also produces no greater than a negligible positive push during withdrawal of the nozzle.

2. The medical valve according to claim 1, wherein the movable member (34) is solid.

3. The medical valve according to claim 1, wherein, the movable member (34) has a substantially static shape transitioning between the first position and the second position.

4. The medical valve according to claim 1, wherein the outlet produces no greater than a negligible drawback or positive push during attachment of the nozzle.

5. The medical valve according to claim 1, wherein the valve further includes a biasing member (36) for biasing the movable member toward the second position.

6. The medical valve according to claim 5, wherein the biasing member (36) is a spring.

7. The medical valve according to claim 6, wherein the biasing member includes an air vent (48).

8. The medical valve according to claim 7, wherein the air vent includes a hydrophobic filter (49).

9. The medical valve according to claim 1, wherein the movable member has an end for contacting the nozzle, the end including at least one of a groove and a protrusion.

10. The medical valve according to claim 1, further comprising at least one seal between the housing and the movable member.

11. The medical valve according to claim 1, wherein, the movable member has an end (56) for contacting the nozzle, the end being swabbable in the second position.

12. The medical valve according to claim 1, wherein the inlet (14) and outlet (16) are longitudinally aligned.

## Patentansprüche

1. Medizinisches Ventil, das Folgendes umfasst:
ein Gehäuse mit einem Inneren (13), das einen Einlass (14) und einen Auslass (16) bildet;
einen Fluidkanal im Inneren; und
ein Fluidkanalbildungsmittel (40) im Inneren, das wenigstens einen Teil des Fluidkanals bildet und eine zentrale Kammer (45) aufweist; und
ein starres, longitudinal bewegliches Element (34), das im Inneren des Gehäuses befestigt ist, wobei sich der Fluidkanal außerhalb des beweglichen Elementes befindet, wobei eine Bewegung des beweglichen Elementes Fluidfluss durch den Fluidkanal zwischen dem Einlass und dem Auslass reguliert,
wobei das bewegliche Element (34) eine erste Position, die Fluidfluss durch den Fluidkanal vom Einlass zum Auslass zulässt, und eine zweite Position hat, die Fluidfluss durch den Fluidkanal vom Einlass zum Auslass verhindert,
der Auslass nur einen vernachlässigbaren Rückzug beim Zurückziehen einer Düse erzeugt,
**dadurch gekennzeichnet, dass** das bewegliche Element in der zentralen Kammer aufgenommen wird und die zentrale Kammer (45) eine Bewegung des longitudinal beweglichen Elementes zwischen der ersten Position und der zweiten Position zulässt, so dass das bewegliche Element (34) in allen Phasen des Öffnungs- und Schließvorgangs einen Teil des Fluidkanals durchläuft, der ein Volumen hat, das in der ersten Position und der zweiten Position im Wesentlichen gleich ist, und der Auslass nur einen vernachlässigbaren positiven Schub beim Zurückziehen der Düse erzeugt.

2. Medizinisches Ventil nach Anspruch 1, wobei das bewegliche Element (34) fest ist.

3. Medizinisches Ventil nach Anspruch 1, wobei das bewegliche Element (34) eine im Wesentlichen statische Form beim Übergang zwischen der ersten Position und der zweiten Position hat.

4. Medizinisches Ventil nach Anspruch 1, wobei der Auslass nur einen vernachlässigbaren Rückzug oder positiven Schub beim Anbringen der Düse erzeugt.

5. Medizinisches Ventil nach Anspruch 1, wobei das Ventil ferner ein Vorspannelement (36) zum Vorspannen des beweglichen Elements zur zweiten Position hin aufweist.

6. Medizinisches Ventil nach Anspruch 5, wobei das Vorspannelement (36) eine Feder ist.

7. Medizinisches Ventil nach Anspruch 6, wobei das Vorspannelement eine Lüftungsöffnung (48) aufweist.

8. Medizinisches Ventil nach Anspruch 7, wobei die Lüftungsöffnung ein hydrophobes Filter (49) aufweist.

9. Medizinisches Ventil nach Anspruch 1, wobei das bewegliche Element ein Ende für einen Kontakt mit der Düse hat, wobei das Ende eine Nut und/oder einen Vorsprung aufweist.

10. Medizinisches Ventil nach Anspruch 1, das ferner wenigstens eine Dichtung zwischen dem Gehäuse und dem beweglichen Element umfasst.

11. Medizinisches Ventil nach Anspruch 1, wobei das bewegliche Element ein Ende (56) für einen Kontakt mit der Düse hat, wobei das Ende in der zweiten Position abgewischt werden kann.

12. Medizinisches Ventil nach Anspruch 1, wobei der Einlass (14) und der Auslass (16) longitudinal fluchten.

## Revendications

1. Valve médicale comprenant :
un logement ayant un intérieur (13) formant une entrée (14) et une sortie (16) ;
un chemin de fluide dans l'intérieur ; et
un moyen formant un chemin de fluide (40) dans l'intérieur, constituant au moins une partie du chemin de fluide et ayant une cavité centrale (45) ; et
un élément rigide mobile dans le sens longitudinal (34) fixé dans l'intérieur du logement, le chemin de fluide étant extérieur à l'élément mobile, le déplacement de l'élément mobile contrôlant le débit entre l'entrée et la sortie via le chemin de fluide,
l'élément mobile (34) ayant une première position qui permet l'écoulement du fluide de l'entrée à la sortie via le chemin de fluide, et une deuxième position qui empêche l'écoulement du fluide de l'entrée à la sortie via le chemin de fluide,
la sortie ne produit pas plus qu'un retour négligeable pendant le retrait d'une buse,
**caractérisée en ce que** l'élément mobile est reçu à l'intérieur de la cavité centrale et la cavité centrale (45) permet le déplacement de l'élément mobile dans le sens longitudinal entre la première position et la deuxième position de telle sorte que, pendant toutes les phases de l'opération d'ouverture et de fermeture, l'élément mobile (34) se déplace sur une partie du chemin de fluide qui a un volume qui est essentiellement le même dans la première position et la deuxième position, et la sortie ne produit aussi pas plus qu'une poussée positive négligeable pendant le retrait de la buse.

2. Valve médicale selon la revendication 1, dans laquelle l'élément mobile (34) est plein.

3. Valve médicale selon la revendication 1, dans laquelle l'élément mobile (34) a une forme essentiellement statique en se déplaçant entre la première position et la deuxième position.

4. Valve médicale selon la revendication 1, dans laquelle la sortie ne produit pas plus qu'un retour ou qu'une poussée positive négligeable pendant la fixation de la buse.

5. Valve médicale selon la revendication 1, la valve comprenant en outre un élément de rappel (36) pour rappeler l'élément mobile vers la deuxième position.

6. Soupape médicale selon la revendication 5, dans laquelle l'élément de rappel (36) est un ressort.

7. Valve médicale selon la revendication 6, dans laquelle l'élément de rappel comprend un évent (48).

8. Valve médicale selon la revendication 7, dans laquelle l'évent comporte un filtre hydrophobe (49).

9. Valve médicale selon la revendication 1, dans laquelle l'élément mobile a une extrémité prévue pour contacter la buse, l'extrémité comprenant au moins une gorge et/ou une saillie.

10. Valve médicale selon la revendication 1, comprenant en outre au moins un joint entre le logement et l'élément mobile.

11. Valve médicale selon la revendication 1, dans laquelle l'élément mobile a une extrémité (56) pour contacter la buse, l'extrémité pouvant être écouvillonnée dans la deuxième position.

12. Valve médicale selon la revendication 1, dans laquelle l'entrée (14) et la sortie (16) sont alignées longitudinalement.
